# EUROPEAN PATENT APPLICATION

(11) **EP 4 588 499 A1**
(43) Date of publication of application: **23.07.2025**
(21) Application number: 24152548.4
(22) Date of filing: 18.01.2024
(51) Int. Cl.: A61M 25/00, B29C 48/09, B29C 48/90, B26D 3/16, B26F 1/24, B29C 48/00

(54) **METHOD FOR PRODUCING CATHETER TUBING**

(71) Applicant: Wellspect AB, 431 21 Mölndal (SE)
(72) Inventor: Utas, Jan, 434 95 Kungsbacka (SE); Claesson, Henrik, 437 38 Lindome (SE)
(74) Representative: Aldridge, Henry Alexander

(57) **Abstract**

The present disclosure relates to a method for producing catheter tubing for a urinary or rectal catheter and a manufacturing system. The method comprises extruding a tubular member by passing a liquid material through a nozzle, the tubular member comprising a rounded outer surface and a central lumen. The method further comprises rotating a first rotatable die around a rotation axis arranged at a longitudinal position downstream of the nozzle, wherein said first rotatable die comprises at least one protrusion and, while said first rotatable die is rotating, pushing said at least one protrusion of the first rotatable die into the tubular member at least a portion of the distance between the outer surface and the central lumen and removing the at least one protrusion from the tubular member, such that the at least one protrusion leaves at least one recess or opening in the tubular member.

## Description

### TECHNICAL FIELD OF THE INVENTION

The present invention relates to a method for producing catheter tubing for a urinary catheter or a rectal catheter, and a manufacturing system.

### BACKGROUND OF THE INVENTION

Different types of catheters are known to be inserted into bodily openings of humans and other mammals. Urinary catheters are for example commonly used for insertion into the urethra for drainage of urine. This type of catheter is typically made from a tubular material having a central lumen and in some manufacturing methods of catheters, the tubular material is first cut to a suitable length, such as 80-200 millimeters for females and 200-400 millimeter for males. Each tube is then closed at a proximal end by forming a rounded tip and an open connector and/or outlet is arranged at the opposite distal end. To form a urinary catheter one or more openings (often referred to as eyelets) are provided in close proximity to the proximal end wherein the one or more openings extend all the way through the tubular material to communicate with the central lumen.

The urinary catheter can then be introduced into the urinary bladder, via the urethra, such that at least one of the one or more openings of the proximal end of the urinary catheter comes into fluid communication with the inside of the urinary bladder. Urine can then flow into the central lumen of the catheter, through the central lumen to the distal end of the catheter and out of the urinary catheter via the connector and/or outlet at the distal end which remains outside the urethra of the patient when in use.

Preferably, the outside of the urinary catheter is coated with a hydrophilic coating which becomes slippery when wetted. This allows users to wet the urinary catheter before use, whereby the urinary catheter becomes very slippery, which makes insertion of the urinary catheter via the urethra more comfortable. In some cases, to make the catheter even more convenient to use the urinary catheter is provided pre-wetted in a state ready to use inside a package.

A problem with existing methods for manufacturing urinary catheters is that the process requires many steps, which makes manufacturing time consuming and labor intensive. Another problem with existing methods for manufacturing urinary catheters is that it is difficult to form openings which smoothly transition into the tubular material surrounding the openings. Smoothly transitioning openings are beneficial since they improve the comfort of the catheter. To form smoothly transitioning openings, manufacturing methods have been proposed involving pushing a heated mandrel into the urinary catheter wherein the heated mandrel has a rounded shape that deforms the material of the urinary catheter to form an opening with rounded edges. Additionally, in EP4048504 ultrasonic punching is proposed as a manufacturing method for forming openings and in EP4046674 a manufacturing method wherein openings are formed in already provided depressions is suggested. However, a drawback with this process is that the manufacturing process requires many steps, which makes them time consuming and costly.

### SUMMARY OF THE INVENTION

It is a purpose of the present invention to overcome at least some of the drawbacks associated with existing methods for manufacturing catheter tubing that can be used to make urinary catheters or rectal catheters.

According to a first aspect of the invention there is provided a method for manufacturing catheter tubing for a urinary catheter or a rectal catheter. The method comprises extruding a tubular member by passing a liquid material through a nozzle, the tubular member comprising a rounded outer surface and a central lumen wherein the tubular member is extruded in a longitudinal direction, and rotating a first rotatable die around a rotation axis arranged at a longitudinal position downstream of the nozzle, wherein the first rotatable die comprises at least one protrusion. The method further comprises, that while the first rotatable die is rotating, pushing the at least one protrusion of the first rotatable die into the tubular member at least a portion of the distance between the outer surface and the central lumen and removing the at least one protrusion from the tubular member, the at least one protrusion leaves at least one recess or opening in the tubular member.

With this method catheter tubing with recesses and/or openings provided on the tubular member may be manufactured in a single extrusion process. The process can be realized as a fully inline process compared to prior solutions requiring multiple separate steps performed at separate locations. It is envisaged that the method can also be used to make complete urinary or rectal catheters in a single inline process.

Additionally, as the first rotational die rotates when the at least one protrusion is pushed into the tubular member, the resulting recess or opening will feature smooth structures and be devoid of sharp edges that otherwise would be present if the recess or opening was formed with drilling, stamping, or the like.

In some implementations, the liquid material is still at an elevated pressure and/or temperature from the extrusion process when the at least one protrusion is pushed into the tubular member. The liquid material may therefore, still be a liquid and/or exhibiting a lower viscosity compared to a when it is completely hardened or solidified. This will in turn allow the recess or opening to become even smoother since the liquid material (while still being at an elevated temperature) is easier to deform smoothly.

With a urinary catheter it is meant any type of urinary catheter including intermittent urinary catheters and indwelling urinary catheters (such as Foley catheters). The urinary catheters are typically used for urinary drainage, but the term "urinary catheter" is interpreted broadly so as to cover all catheters inserted via the urethra and which comprise at least one lumen. For example, urinary catheters comprise catheters used in urology, continence care, urinalysis and/or used in uroflowmetric measurements.

With "catheter tubing" it is meant tubes that can be used to form a complete urinary catheter or rectal catheter. In some implementations, the catheter tubing will be cut into suitable lengths, and e.g. provided with closed rounded tip, to form a complete urinary or rectal catheter.

With a rectal catheter it is meant any type of rectal catheter that is configured to be inserted into the rectum of a user and which comprises at least one central lumen and at least one opening communicating with the central lumen. Examples of rectal catheters include rectal catheters for transanal irrigation (TAI) that are used to irrigate the bowel.

While a rectal catheter may be shorter and having a larger outer diameter compared to a urinary catheter the two types of catheters are for the purposes of the manufacturing method and system of the present disclosure very similar, wherein both types of catheters benefit from smoothly transitioning openings without sharp edges which facilitates insertion comfort and reduces the risk of the user being injured when inserting the urinary/rectal catheter.

In general, any features described in connection with a method or system for producing catheter tubing for a urinary catheter may also be present in a method or system for producing catheter tubing for a rectal catheter, or vice versa.

The material which is extruded from the nozzle may be a molten material which is liquid. For example, the material is a high viscosity liquid such as a melted thermoplastic material e.g. a thermoplastic polymer or thermoplastic elastomer. The material may also be a viscoplastic material which is a solid material below a critical level of stress and/or heat but flows like a viscous liquid at greater values of stress and/or heat. Additionally, it is envisaged that the material may be viscoelastic material, i.e. a material which exhibits both elasticity and viscosity.

In some implementations, to facilitate smooth deformation of the material the material is heated at the time when the at least one protrusion is inserted and removed. Heating may decrease the viscosity of the material such that it flows to form smooth structures.

In some implementations, the method for producing catheter tubing further comprises pushing the at least one protrusion of the first rotatable die into the tubular member a portion of the distance between the outer surface and the at least one central lumen and removing the at least one protrusion from the tubular member, such that the at least one protrusion leaves a recess in the outer surface of the tubular member wherein the method further comprises providing an opening inside the recess, the opening extending to communicate with the central lumen.

That is, the at least one protrusion is used to only form a smooth recess in the tubular member.

The formation of the opening, inside the recess, may be performed in any suitable manner. For example, the opening is formed with another rotatable die having a protrusion that forms the opening inside the recess. As another example, the opening inside the recess is formed using drilling, etching, punching or stamping. The opening may also be formed using laser cutting or water-jet cutting. Since the footprint of the opening is smaller than the recess, the smooth structures of the recess will still be arranged closer to the outer surface while any potential sharp edges formed by formation of the opening will be located comparatively further away from the outer surface.

A benefit with only forming the recess, and providing the opening inside the recess later, is that it may be easier to coat the catheter tubing with additional coating layers. In some implementations, especially for catheter tubing for urinary catheter, the final catheter is coated with a hydrophilic coating and by providing only the recess first, it is possible to coat the urinary catheter with the hydrophilic coating without the coating material reaching the central lumen.

In some implementations, the method for producing catheter tubing further comprises pushing the at least one protrusion of the first rotatable die into the tubular member the full distance between the outer surface and the at least one central lumen and removing the at least one protrusion from the tubular member, such that the protrusion leaves an opening in the tubular member, the opening communicating with the central lumen.

Hereby, the at least one protrusion may extend sufficiently far from the circumferential surface such that when it is pushed into the tubular member it goes all the way through to the central lumen, thereby leaving an opening upon removal. The opening will feature smooth structures that transition smoothly into the outer surface while still enabling fluid communication between the central lumen and the outside of the tubular member. An opening in the tubular member may be used as an eyelet in a catheter realized from the tubular member. The eyelet may be used for drainage of urine or stool via the central lumen, injection of a fluid (e.g. water) into the bladder or bowel or injection of fluid (e.g. water or air) into an inflatable retention member

A benefit with forming the opening with the rotatable die is that the opening may be formed primarily by pushing material away from opening rather than actively removing material (as is the case for stamping or drilling). This overcomes the risk of traditional methods for forming an opening wherein removed material comes loose and gets trapped inside the central lumen of the catheter. Detecting this type of removed material may be challenging which poses a risk that the material remains inside the central lumen when a user uses the catheter. That is, the method for producing catheter tubing of the present invention may not generate any residual material at all wherein the risk of left-over residual remaining inside the central lumen is reduced.

It is understood that in some implementations a plurality of protrusions is arranged on the rotatable die wherein it is envisaged that one protrusion may form a recess and one protrusion may form an opening. Alternatively, each protrusion of the plurality of protrusions may form an opening or each protrusion of the plurality of openings may form a recess.

In some implementations, the method further comprises rotating a second rotatable die around a second rotation axis arranged at a second longitudinal position downstream of the nozzle, wherein the second rotatable die comprises at least one protrusion and, while the second rotatable die is rotating, pushing the at least one protrusion of the second rotatable die into the tubular member at least a portion of the distance between the outer surface and the central lumen. The method further comprises removing the at least one protrusion of the second rotatable die from the tubular member, such that each at least one protrusion leaves at least one second recess or opening in the tubular member.

Accordingly, multiple rotatable dies may be arranged downstream of the nozzle where each rotatable die comprises at least one protrusion forming a respective recess or opening in the tubular member. In this way, recesses and/or openings may be formed in different circumferential sectors on the tubular member. As the tubular member is intended to be used as a urinary or rectal catheter, multiple rotatable dies may be used to form openings and/or recesses going circumferentially around the tubular member which in turn will form eyelets distributed circumferentially around the urinary catheter that provides e.g. efficient urine drainage and/or irrigation in multiple directions.

For example, the first and second rotatable dies are arranged on opposite sides of the tubular member such that the first recess or opening and second recess or opening are formed on opposite sides of the tubular member.

In some implementations, the method for producing catheter tubing further comprises passing the tubular member over a support structure, the support structure being arranged in the central lumen and extending at least to the first longitudinal position downstream of the nozzle where the protrusion is pushed into the tubular member.

That is, the support structure functions as an anvil that upholds the structure of the central lumen when the at least one protrusion is pushed into the tubular member. The support structure thereby prohibits the tubular member from deforming or collapsing. When the at least one protrusion is pushed into the tubular member there is a risk that the tubular member, especially when it is still heated by the extrusion process, collapses or becomes deformed. With the support structure, the desirable rounded outer shape of the tubular member, as well as the shape of the central lumen, can be maintained.

Additionally or alternatively to utilizing a support structure, the method for producing catheter tubing may comprise injecting a fluid inside the central lumen of the tubular member to increase the pressure inside the central lumen.

The fluid is typically a gas, such as air, and injection of the fluid creates a positive pressure inside the central lumen which adds rigidity and supports the central lumen such that it does not collapse when the at least one protrusion is pushed into the material.

Additionally or alternatively, the method for producing catheter tubing comprises, downstream of each rotatable die, inserting the tubular member into a tube shaped chamber, the tube shaped chamber having an inner diameter which is greater than or equal to an outer diameter of the tubular member and providing a negative pressure inside the tube shaped chamber to exert an expanding force on the tubular member.

A tube shaped chamber with a negative pressure is therefore another alternative for facilitating maintaining the shape of the tubular member after extrusion. The negative pressure will in effect pull the tubular member outwards which thereby prevents the tubular member from deforming or collapsing. As the chamber is shaped like a tube, such as a cylindrical surface, having an inner diameter which is greater than or equal to the outer diameter of the tubular element, the negative pressure will exert an even force over the tubular member which facilitates maintaining the rounded shape even when protrusions are pushed into the material and/or as the liquid material solidifies. The process of shaping the extruded tubular member may be referred to as a sizing process or profile calibration process which as such is known in the field of extrusion. Accordingly, the tube shaped chamber may be referred to as a calibration/sizing pipe or calibration/sizing tube.

It is understood that using a support structure, injecting a fluid into the central lumen and passing the tubular member through a negatively pressurized tube shaped chamber are all alternatives that can be combined or implemented separately.

In some implementations, the first rotatable die is circular and has a circumference equaling a repetition length for the forming of the at least one recess or opening, wherein the method further comprises rotating the first rotatable die such that a tangential velocity at the periphery of the first rotatable die equals an extrusion velocity of the tubular member.

When the circumference of the rotatable die equals the repetition distance the tangential speed at the peripheral surface may equal the extrusion velocity. This in turn allows the rotatable die to e.g. be rotated by the tubular member being extruded. For instance, it is envisaged that the rotatable die is in contact with the tubular member and rotated by the tubular member being continuously in contact with the tubular member.

In some implementations, at least the first rotatable die is linearly displaceable at least along a transverse axis perpendicular to the longitudinal axis wherein the method further comprises moving the first rotatable die transversely towards the tubular member, prior to or during the pushing of the at least one protrusion of the first rotatable die into the tubular member and moving the first rotatable die transversely away from the tubular member, during or after removal of the at least one protrusion of the first rotatable die from the tubular member.

By using a linearly displaceable rotatable die, the circumference of the rotatable die and/or the rotational speed of the rotatable die can be decoupled from the repetition distance. This may e.g. allow utilization of a rotatable die that is smaller in diameter, and/or which has a shape other than circular, which in turn may allow the rotatable die to be placed closer to the nozzle so as to push its protrusion into the tubular member at a location closer to the nozzle where the liquid material is still at an elevated temperature.

In some implementations, the first rotatable die comprises a plurality of protrusions forming a plurality of recesses and/or openings.

Accordingly, a plurality of protrusions may be arranged on the rotatable die to form multiple corresponding recesses and/or openings on the tubular member. As the recess(es) and/or opening(s) are used to form e.g. eyelets in a catheter, the recesses and/or openings may be of the size typically used for urinary catheters and/or arranged with a spatial density typically used for urinary catheters. For example, between 2 and 12 recesses and/or openings may be provided on the tubular member. Additionally, many more recesses and/or openings may be provided on the tubular member such as more than 12, at least 50, at least 100 or at least 150. In some implementations, the recesses and/or openings may be provided on different sides of the tubular member by different rotatable dies. For example, each rotatable die may comprise between 1 and 6 protrusions wherein each protrusion creates a recess or opening.

Rectal catheters typically have larger and/or fewer openings (such as one opening, two openings or four openings) for fluid injection but it is envisaged that more than ten openings can be used also for rectal catheters.

The total opening area of all openings (formed with the at least one protrusion of the rotatable die, or separately) is preferably at least 50% of the cross-sectional area of the central lumen, at least 70% of the cross-sectional area of the central lumen or at least 100% of the cross-sectional area of the central lumen. Typically, the total opening area of all openings is between 100% and 150% of the cross-sectional area of the central lumen. Adding more and/or larger openings may be desirable to e.g. increase the urine flow rate of a urinary catheter while also making the catheter able to maintain a high flowrate even when one or more openings are obstructed. On the other hand, the opening area of the central lumen puts a limit on how large the urine flow rate or fluid injection rate can be, and it is generally desirable to avoid incorporating openings having a too large total opening area as this could compromise the structural stability of the catheter, e.g. making it more prone to kinking or breaking. To this end, the total area of the openings formed on a part of the tubular member intended to be used as one catheter is below 300% of the central lumen cross-sectional area, below 250% of the central lumen cross-sectional area or below 200% of the central lumen cross-sectional area.

It is often desirable to place the recess(es) and/or opening(s) close together over a longitudinal distance of the tubular member that will be close to the insertion end of the urinary or rectal catheter. For example, the plurality or recesses and/or openings are distributed over a longitudinal distance that is smaller than 10 cm, smaller than 8 cm or smaller than 6 cm.

In some implementations, the method for producing catheter tubing further comprises cutting the tubular member into a tubular member section, the section having at least one opening or recess, and forming a closed tip at one end of the tubular member section.

Hereby, it is evident that complete urinary or rectal catheters with recesses and/or openings having smooth edges and structures can be realized with comparatively few steps in an efficient manufacturing method. Optionally, the method further comprises arranging a connector at the end opposite of the closed tip.

In some implementations, an outer diameter of the tubular member is smaller than 10 mm, preferably smaller than 8 mm and most preferably smaller than 7 mm, if the tubular member is to be used a urinary catheter. On the other hand if, the tubular member is to be used as a rectal catheter the outer diameter is preferably at least 5 mm, at least 10 mm or at least 12 mm. For example, the outer diameter of the tubular member when it is intended to be used as rectal catheter is between 5 mm and 15 mm.

Since the tubular member is to be used as a urinary or rectal catheter, its outer diameter preferably constitutes a regular urinary or rectal catheter diameter.

According to a second aspect of the invention there is provided a manufacturing system for producing catheter tubing for urinary or rectal catheters. The manufacturing system comprises a nozzle, configured to extrude a liquid material to form a rounded tubular member having a rounded outer surface and a central lumen wherein the tubular member is extruded in a longitudinal direction, and a first rotatable die, the first rotatable die comprising a smooth cylindrical peripheral surface and at least one protrusion arranged on the peripheral surface wherein the rotatable die is configured to rotate around a rotational axis at a longitudinal position downstream of the nozzle and wherein all protrusions are confined to a region covering at most 20% of the peripheral surface of the rotational die.

With a region covering at most 20% of the peripheral surface it is meant the angle occupied by the region as measured around the rotational axis does not occupy more than 20% of the full 2π radians spanning the whole rotatable die.

Optionally, all protrusions are confined to a region covering at most 15%, or at most 10%, of the peripheral surface of the rotational die.

Since urinary or rectal catheters typically have eyelets confined to only a small portion of the total length of the urinary or rectal catheter, the rotatable die may be realized with protrusions spanning only a small portion of the total peripheral surface.

According to a third aspect of the invention there is provided a manufacturing system for producing catheter tubing for urinary or rectal catheters. The manufacturing system comprises a nozzle, configured to extrude a (e.g. heated) liquid material into a rounded tubular member having a central lumen wherein the tubular member is extruded in a longitudinal direction and a first rotatable die, the first rotatable die comprising at least one protrusion and arranged to rotate around a rotational axis at a longitudinal position downstream of the nozzle such that while the first rotatable die is rotating, it is pushing the at least one protrusion of the first rotatable die into the tubular member along at least a portion of the distance between the outer surface and the central lumen, and removing the at least one protrusion from the tubular member, such that the protrusion leaves at least one recess or opening in the tubular member.

The invention according to the second and third aspect features the same or equivalent benefits as the invention according to the first aspect. Any functions described in relation to the method, may have corresponding features in a manufacturing system or vice versa.

The person skilled in the art realizes that the present invention by no means is limited to the preferred embodiments described above. On the contrary, many modifications and variations are possible within the scope of the appended claims. For example, while one or two rotatable dies may be used, it is further envisaged that more than two rotatable dies can be used, each comprising one or more protrusions.

In another flavor of the present invention the liquid material is a light-activated resin and/or a thermo-activated resin. Examples of light-activated resins are photopolymers that form crosslinks when exposed to certain types of light (such as UV or IR light). Examples of thermo-activated resins are thermosetting polymers which undergo crosslinking when exposed to heat. When the material is a light-activated or thermo-activated resin it may no longer be necessary to heat the material prior to, or during, extrusion. For example, the light-activated or thermo-activated resin may be soft, deformable and extrudable at room temperature. The light-activated or thermo-activated resin is made solid by a subsequent curing process after the recess and/or opening with recess has been formed. The curing process comprises the step of irradiating or heating the tubular member after (i.e. downstream of) the at least one protrusion has removed from the tubular member such that the light-activated or thermo-activated resin or soft solid material cures by forming crosslinks.

Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements.

### BRIEF DESCRIPTION OF THE DRAWINGS

The present invention will be described in more detail with reference to the appended drawings, showing currently preferred embodiments of the invention.
Figure 1 shows a manufacturing system for producing catheter tubing with a rotatable die according to some implementations.
Figure 2 shows an alternative manufacturing system for producing catheter tubing with two opposing rotatable dies according to some implementations.
Figure 3a-c shows a cross-sectional view of the tubular member when the protrusion of a rotatable die is pushed into and removed from the tubular member to form a recess.
Figure 3d-e shows a cross-sectional view of the tubular member when a mandrel is introduced into the tubular member for forming an opening through an already provided recess in the tubular member.
Figure 4 shows a cross-sectional view of the tubular member where an opening has been formed by a protrusion on the rotatable die.
Figure 5a-d illustrates schematically some post-processing steps performed on the tubular member according to form a complete urinary or rectal catheter according to some implementations.
Figure 6a-c illustrates an exemplary shape of the recess and/or opening formed in the tubular member according to some implementations.
Figure 7 is a flow-chart illustrating a method for producing catheter tubing for a urinary or rectal catheter, according to some implementations.
Figure 8a-e show exemplary rotatable dies with varying arrangements of the protrusion(s).
Figure 9a-b illustrates the relationship between the rotatable die circumference and the repetition distance according to some implementations.
Figure 10 shows a rotatable die which is linearly moveable along the transverse direction according to some implementations.
Figure 11 shows a rotatable die with a circumference that is smaller than the repetition distance according to some implementations.
Figure 12 shows a cross-sectional view of a tubular member and four rotatable dies arranged to engage the tubular member form different directions.
Figure 13 shows a support structure arranged inside the central lumen according to some implementations.
Figure 14 shows a nozzle for injecting a fluid into the central lumen according to some implementations.
Figure 15 shows a tube-shaped chamber for maintaining a negative fluid pressure that the tubular member can be passed through.

### DETAILED DESCRIPTION OF CURRENTLY PREFERRED EMBODIMENTS

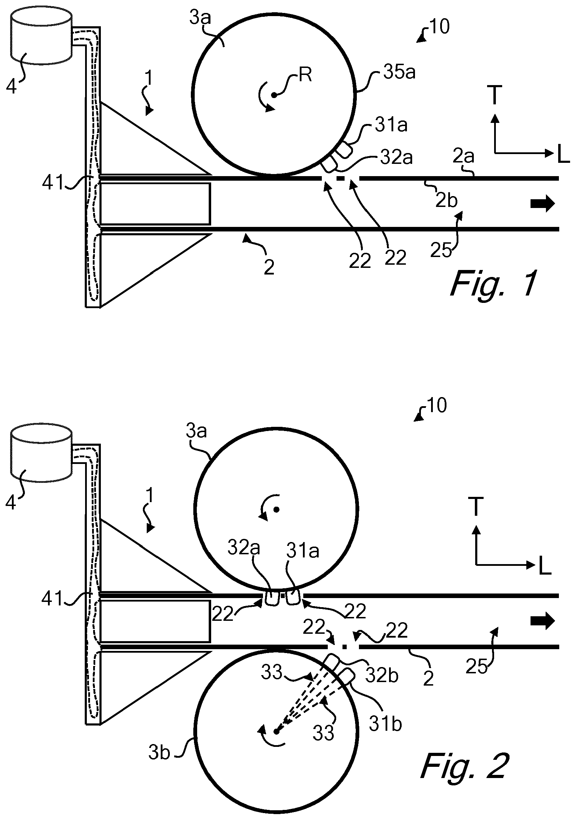

Fig. 1 shows schematically a manufacturing system 10 for producing catheter tubing in the form of an elongated tubular member 2. The catheter tubing can be used to form one or more urinary or rectal catheters. The manufacturing system 10 utilizes an extrusion process and the manufacturing system 10 comprises one or more rotatable dies 3a wherein each rotatable die 3a is configured to form an opening 22 and/or a recess in the tubular member 2 while the extruded tubular member is still heated, softened or exhibiting a reduced viscosity after being extruded.

The manufacturing system 10 comprises an extrusion nozzle 1 configured to extrude a tubular member 2.

The tubular member 2 is rounded. For example, the outer shape of the cross-section of the tubular member 2 is substantially oval, elliptical or circular. That is, the tubular member 2 can be said to be a hollow, oval, elliptical or circular cylinder.

The extruded tubular member 2 is hollow and comprises at least one central lumen 25 that extends on the inside of the tubular member 2. The cross-section of the central lumen 25 will typically be of the same shape as that of the outer surface of the tubular member 2. In one example, the tubular member 2 has a circular cross-section and the central lumen also has a circular cross-section. On the other hand, the cross-sectional shape of the outer surface of the tubular member may be different from that of the central lumen 25. For example, the cross-section of the outer surface of the tubular member is elliptical whereas the cross-section of the central lumen is circular, or vice versa.

Optionally, it is envisaged that the extrusion nozzle 1 may be configured to extrude a tubular member 2 with two or more separate lumens 25. Such tubular members 2 may e.g. be used to form urinary or rectal catheters that have a first lumen for discharge of urine or stool or for fluid injection, and a separate, second lumen for supplying fluid to an inflatable retention member. An example of a urinary catheter with two lumens and an inflatable retention member is a Foley catheter typically used as an indwelling urinary catheter.

Since the extrusion nozzle 1 is configured to extrude a hollow and rounded tubular member 2 the nozzle opening may be shaped like an oval, elliptical or circular ring. For example, the nozzle opening is shaped like a circular ring, having a greater outer radius r₁ and lesser inner radius r₂, wherein the difference r₁ - r₂ determines the wall thickness of the tubular member 2.

A (e.g. heated) liquid material 41 passes through the extrusion nozzle 1 and the extrusion nozzle 1 extrudes the tubular member 2 which exits in the longitudinal direction L. The liquid material 41 may be supplied from a reservoir 4 and the liquid material 41 is heated either prior to being passed through the nozzle 1, while it passes through the nozzle 1 or both. For example, the extrusion nozzle 1 may be a heated nozzle 1 that heats the liquid material 41 and/or melts a solid material that when melted forms the liquid material 41.

There are many types of materials, mixtures and blends that can be used as the liquid material 41. In general, any liquid material 41 used in an extrusion process is solid at room temperature (around 20°C) and melts to form a liquid at elevated temperatures (usually at temperatures exceeding 100°C) to form a high viscosity fluid. The liquid material 41 may comprise one or more plastic materials, and in particular polymer and/or thermoplastic materials, such as materials conventionally known for use as urinary or rectal catheter shafts and the like. For example, the liquid material is melted thermoplastic material. The liquid material 41 may e.g. comprise one or more of polyurethane, nylon, polyolefins, silicone, polyether block amide etc. Additional examples of liquid materials 41 are silicone rubber, latex rubber, and thermoplastic elastomers (TPE). Blends of different materials may also be used, or layered structures where layers of different materials are sandwiched over each other, e.g. by co-extrusion. Co-extrusion may e.g. be realized with a nozzle 1 having an outlet forming two concentric rings, a smaller ring forming an inner layer during extrusion and a larger ring arranged outside the smaller ring forming the outer layer during extrusion. In this example, each concentric ring-shaped outlet is fed by a respective liquid material 41.

The solid material that is melted to form the liquid material may be provided in the form of slab, a thread filament, a powder and/or as beads or pellets. The solid material is then melted to form the liquid material 41 that is extruded out of the nozzle 1. To facilitate extrusion and/or to achieve certain properties in the final extruded product one or more additives may also be added to the solid material or the liquid material 41.

If the tubular member 2 is to be used to form a urinary catheter, the outer diameter of the tubular member 2 is preferably less than 10 mm, preferably than 8 mm and most preferably less than 7 mm. Commonly, urinary catheters used by male and female users have a diameter of 6 mm or less, such as 5.5 mm or less. Most urinary catheters are however larger than 1.5 mm. Hereby, the outer diameter of the tubular member 2 is typically between 1.5 mm and 6 mm. Depending on the properties of liquid material 41 and/or whether any support structure is used inside the central lumen during extrusion it is envisaged that the tubular member 2 may exhibit some shrinkage during curing or solidifying. However, it is appreciated by the person skilled in the art that by configuring the nozzle outlet diameter any shrinkage can be compensated such that the tubular shaft 2, after curing, has an outer diameter less than 10 mm, such as in the range 1.5 mm to 6 mm.

If the tubular member 2 is to be used to form a rectal catheter, the outer diameter of the tubular member is typically larger such as at least 8 mm, at least 10 mm or at least 12 mm.

As mentioned above, the manufacturing system 10 comprises a first rotatable die 3a having at least one protrusion 31a, 32a protruding out from a circumferential surface 35a of the rotatable die 3a. The first rotatable die 3a, is configured to rotate around a rotational axis R and in the embodiment depicted in fig. 1 the rotational axis R is perpendicular longitudinal direction L. However, this is just an example, and it is envisaged that the rotational axis R may be oriented differently, e.g. parallel to the longitudinal axis.

The first rotatable die 3a is further configured to, when the rotatable die 3a rotates, drive the at least one protrusion 31a, 32a into the tubular member such that the at least one protrusion 31a, 32a enters into the tubular member 2 at least a portion of (e.g. only a portion of, or fully through) the distance from an outer surface 2a of the tubular member 2 to an inner surface 2b defining the central lumen 25.

The depth into which the at least one protrusion 31a, 32a is pushed will determine the depth of the recess and whether a through-going opening also is formed inside the recess. By configuring the rotatable die 3a differently (for example by using a larger or smaller diameter rotatable die 3a, moving the axle around which the rotatable die rotates relative the tubular member 2 and/or using a protrusion(s) which extend further from the circumferential surface 35a) the depth of the recess and whether a through-going opening 22 is formed can be controlled. For example, with the rotatable die 3a arranged as shown in fig. 1, the resulting recess may be elongated along the tubular member 2 extending along the longitudinal direction L.

On the other hand, if the rotatable die 3a is arranged with the rotational axis R parallel to the longitudinal direction L the recesses formed by the one or more protrusions when the rotatable die rotates will extend transverse across the tubular member 2. Depending on the angular velocity of the rotatable die 3a with respect to the extrusion speed the resulting recess may extend at different angles with respect to the longitudinal direction.

When the protrusion 31a, 32a has been pushed into the tubular member, the rotatable die 3a continues to rotate so as to remove the at least one protrusion 31a, 32a from the tubular member 2. When the at least one protrusion 31a, 32a is removed, it leaves behind at least one of: a recess in the outer surface of the tubular member 2 and a recess with an opening 22 extending into the central lumen 25 of the tubular member 2.

The at least one protrusion 31a, 32a enters into the tubular member 2 at a position downstream of the nozzle 1. Preferably, the rotatable die 3a is configured to push the at least one protrusion 31a, 32a while the extruded tubular member 2 is still at an elevated temperature after the extrusion, whereby the tubular member 2 may still be soft and/or only partially cured, as this allows the liquid material to flow and deform gently when the at least one protrusion 31a, 32a is pushed into the tubular member 2 and removed. A result of this is that the resulting recess or opening 22 may feature smoother, less sharp, edges, which in turn enables formation of a urinary or rectal catheter that is more comfortable to use.

In some implementations, a second rotatable die 3b is further provided in the manufacturing system 10 as depicted in fig. 2. The second rotatable die 3b comprises at least one protrusion 31b, 32b configured to be pushed into, and subsequently removed from, the tubular member 2. The second rotatable die 3b is however arranged to push the at least one protrusion 31b, 32b into a different (angular) sector of the tubular member 2, compared to the first rotatable die 3a. The different sectors on the tubular member 2, which the first and second rotatable die 3a, 3b are configured to interact with, are angularly displaced around the circumference of the tubular member 2. For example, in fig. 2 the first rotatable die 3a and second rotatable die 3b are arranged on opposite sides of the tubular member 2 whereby the rotatable dies 3a, 3b each form at least one recess, or recess with opening 22, on opposite sides of the tubular member 2. Accordingly, the rotatable dies 3a, 3b may be arranged in a north-south arrangement with the two regions being separated by 180° when seen in the longitudinal direction. In general, however, the first and second rotatable die 3a, 3b may be arranged so as to interact with sectors on the tubular member 2 separated by an arbitrary angle, such as 90° (forming a north-west or north-east arrangement) or 45°.

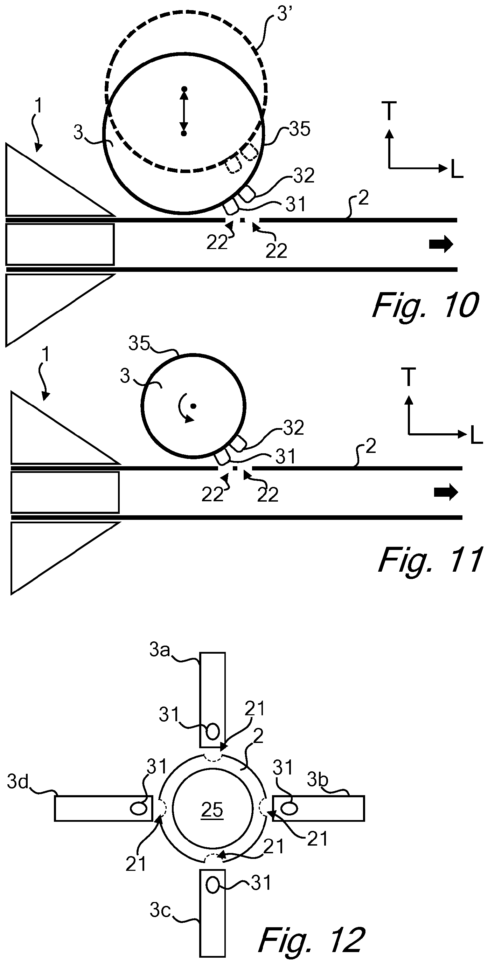

It is also envisaged that more than two rotatable dies 3a, 3b can be used, as will be described below, in connection to fig. 12.

When more than one rotatable die 3a, 3b is used, it is envisaged that the rotatable dies 3a, 3b can be rotated in phase or out of phase with each other. A result of this may be that the recess, or recess with opening 22, formed in each sector will either be at the same longitudinal position along the tubular member or offset at different longitudinal positions. It is envisaged that the phase of the rotation of the rotatable dies 3a, 3b, and the number and/or shape of each at least one protrusion 31a, 32a, 31b, 32b can be varied to form a wide range of different patterns. Similarly, by varying the size and shape of each protrusion, the shape and depth of the corresponding recess, or recess with opening 22, can be varied.

In some implementations, since the tubular member 2 is to be used e.g. as a urinary catheter, the recesses and/or recesses with openings 22 are arranged in a longitudinal eyelet region having a longitudinal extension of less than 10 cm, less than 8 cm or less than 6 cm. This longitudinal region will be close to the insertion end of the urinary catheter that will enter into the urinary bladder, whereby urine can drain via eyelets formed in the recess or formed by the openings in the longitudinal eyelet region. If the tubular member 2 is to be used as a rectal catheter, irrigation fluid is instead injected into the rectum via the central lumen 25 and the eyelets formed in the catheter.

Fig. 2 also shows dashed lines indicating the angular region 33 occupied by each of the protrusions of the second rotatable die 3b. As seen, the protrusions 31b, 32b do not occupy more than 20% of the full 2π radians covering the whole rotatable die. In some implementations, the protrusions do not occupy more than 15% of the full 2π radians spanning the whole rotatable die 3b or not more than 10% of the full 2π radians spanning the whole rotatable die 3b. This sparse placement of the one or more protrusions is enabled by the urinary or rectal catheters typically having openings distributed over less than 20% of their total longitudinal length.

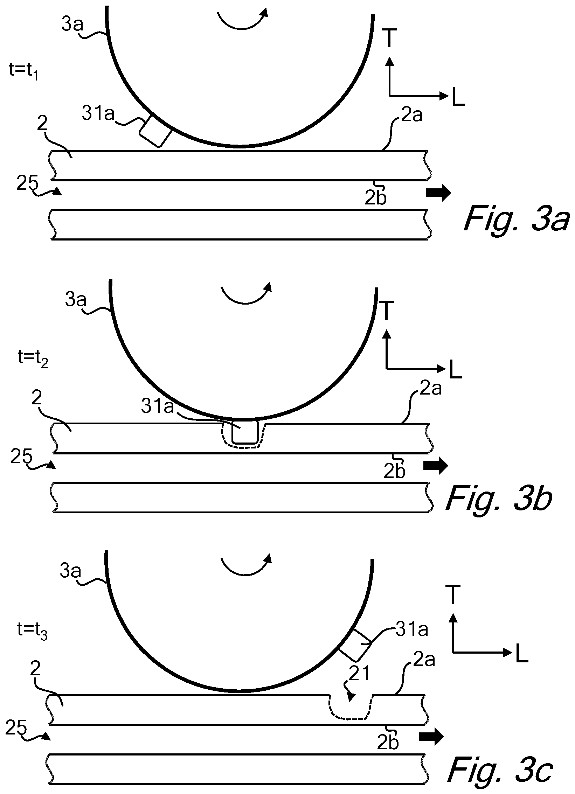

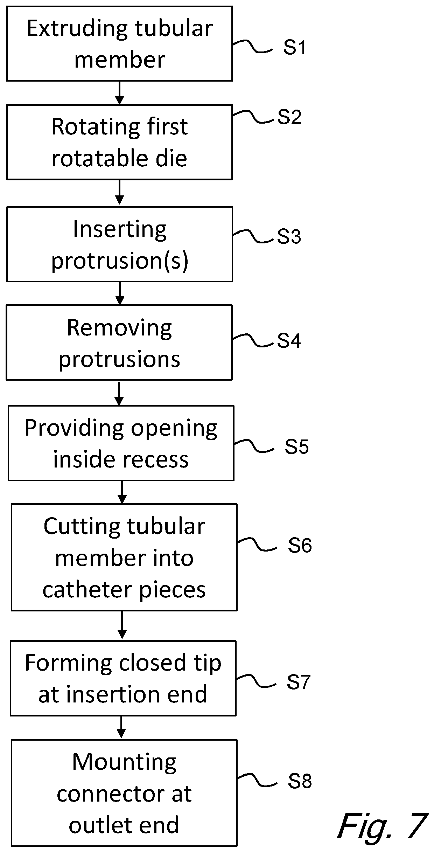

Figs. 3a-3c show close-up views of how a protrusion 31a of a rotatable die 3a is pushed into and removed from the tubular member 2 when the rotatable die 3a rotates. With further reference to the flow chart in fig. 7, a method for producing catheter tubing for a urinary or rectal catheter will now be described.

At step S1, the tubular member 2 is extruded. The tubular member 2 comprises a central lumen 25 and is extruded in the longitudinal direction L (to the right in fig. 3a). At step S2 the rotatable die 3a is rotated. For example, at step S2 the position of the rotatable die 3a relative the tubular member 2 may be as shown in fig. 3a illustrating a cross-sectional view of the tubular member 2 and the rotatable die at a first point in time t₁ before the recess, or recess with opening, has been formed. The rotatable die 3a is rotating counterclockwise in this example and the protrusion 31a approaches the outer surface of the tubular member 2 along the transverse axis T that is perpendicular to the longitudinal direction L.

At step S3 the protrusion 31a is pushed into the tubular member 2 as shown in fig. 3b. For example, step S3 occurs at a second point in time t₂, the second point in time t₂ being later than the first point in time t₁. At t₂ the tubular member 2 has been extruded further and moved along the longitudinal direction L compared to t₁ shown in fig. 3a. At the same time, the rotatable die 3a has continued to rotate in the counterclockwise direction whereby the at least one protrusion 31a has been pushed into the tubular member 2. More specifically, the protrusion 31a has been pushed into the tubular member from the outer surface 2a at least partially along the transverse axis T. The protrusion 31a forms a recess in the outer surface 2a of the tubular member 2 and in this configuration, the protrusion 31a does not form an opening, only a recess, since the protrusion 31a does not pass all the way through to reach the inner surface 2b delimiting the central lumen 25. However, by e.g. using a protrusion 31a which protrudes further from the circumferential surface of the rotatable die 3a or if the tubular member 2 is realized with thinner material, it is possible to also form a through-going opening with the protrusion 31a.

At step S4 the protrusion is removed from the tubular member and fig. 3c shows the tubular member 2 and rotatable die at a third point in time t₃, the third point in time t₃ being later than the second point in time t₂. At t₃ the rotatable die 3a has continued to rotate counterclockwise from its position at t₂. Effectively this withdraws the protrusion 31a from being inserted into the tubular member and leaves a recess 21 in the outer surface 2a of the tubular member 2. During withdrawal, the protrusion 31a moves at least partially along the transverse axis T.

The rotatable die in figs. 3a-c comprises a single protrusion 31a, however it is envisaged that two or more protrusions 31a may be provided, whereby the tubular member 2 will be provided with two or more openings, or recesses with openings, each opening or recess corresponding to one protrusion 31a.

The recess 21 formed by this process may feature very smooth edges, especially in proximity to the outer surface 2a. This will facilitate comfortable insertion when the tubular member is used as a rectal or urinary catheter. To form an opening inside the recess, leading to the central lumen 25, after a recess has been formed with the rotatable die, various processes can be used. For example, an opening can be formed by drilling, etching, melting, or stamping the material inside the recess 21. Preferably, the footprint of the opening is smaller than the footprint of the recess formed in the outer surface 2a. This enables the smooth edge surfaces closest to the outer surface 2a to be preserved.

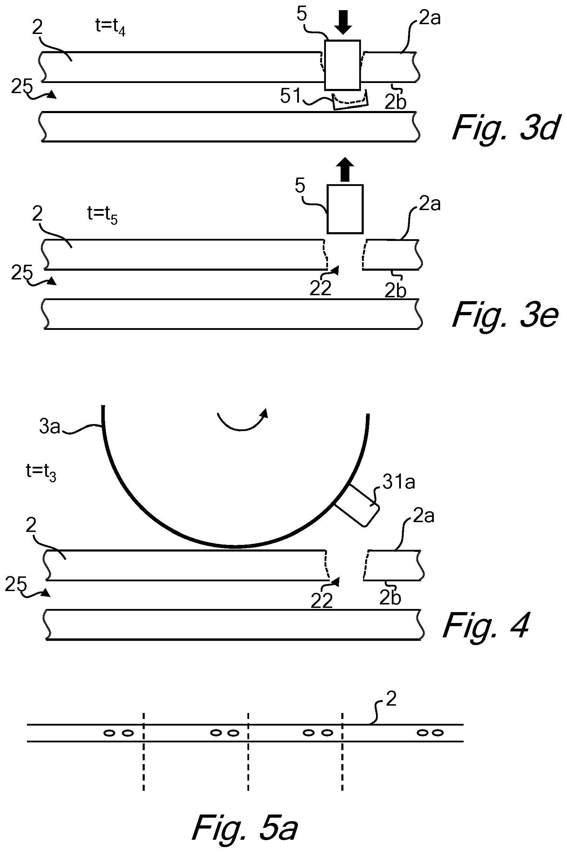

If only a recess 21 has been formed, the method may further comprise step S5 of providing an opening 22 in at least one recess. Figs. 3d and 3e show an example of how an opening 22 can be provided in an already provided recess using a mandrel. At time t₄, which is later than time t₃, a mandrel 5 is inserted into the recess 21 to form an opening 22 inside the recess. A piece 51 of the tubular member 2 comes loose and is removed. For example, the piece 51 is flushed out through the central lumen 25 or it is envisaged that the mandrel 5 is hollow and captures the piece 51. Turning to fig. 3e showing the setup at time t₅, which is later than time t₄, it is seen how the mandrel 5 is removed leaving an opening 22, inside the recess through the tubular member 2, wherein the opening 22 communicates with the central lumen 25. The smooth edges close to the outer surface 2a formed by the at least one protrusion on the rotatable die remain and the opening 22 is formed fully inside the recess.

As illustrated in fig. 4, it is envisaged that the recess and opening is formed at the same time. That is, step S5 may occur at same time as steps S3 and S4. Forming a recess and opening at the same time may be realized with rotatable die 3a shown in fig. 4. Comparing fig. 4 to fig. 3c reveals that the only difference is that the protrusion 31a in fig. 4 extends further from the circumferential surface of the rotatable die 3a compared to the protrusion 31a in fig. 3c. A consequence of this is that that the protrusion 31a in fig. 4 is sufficient to penetrate all the way through the tubular member to form an opening 22 directly, extending from the outer surface 2a to the inner surface 2b, delimiting the central lumen 25. Due to the protrusion 31a being pushed into the tubular member 2 while the rotatable die rotates, the opening 22 will inherently be surrounded by a recessed region of smooth edges that transitions smoothly to an opening.

The openings 22 formed in the tubular member will serve as eyelets when the tubular member is used as a urinary or rectal catheter. Accordingly, the total opening area of all openings 22 is preferably in the range of 50% - 300%, such as in the range of 100% - 250% or 120% - 150%, of the cross-sectional area of the central lumen 25. The total opening area may be formed by a large number of smaller openings or a fewer number of larger openings. Typically, the number of openings is between 1 and 250, such as between 2 and 150. In some implementations, the number of openings is between 2 and 12.

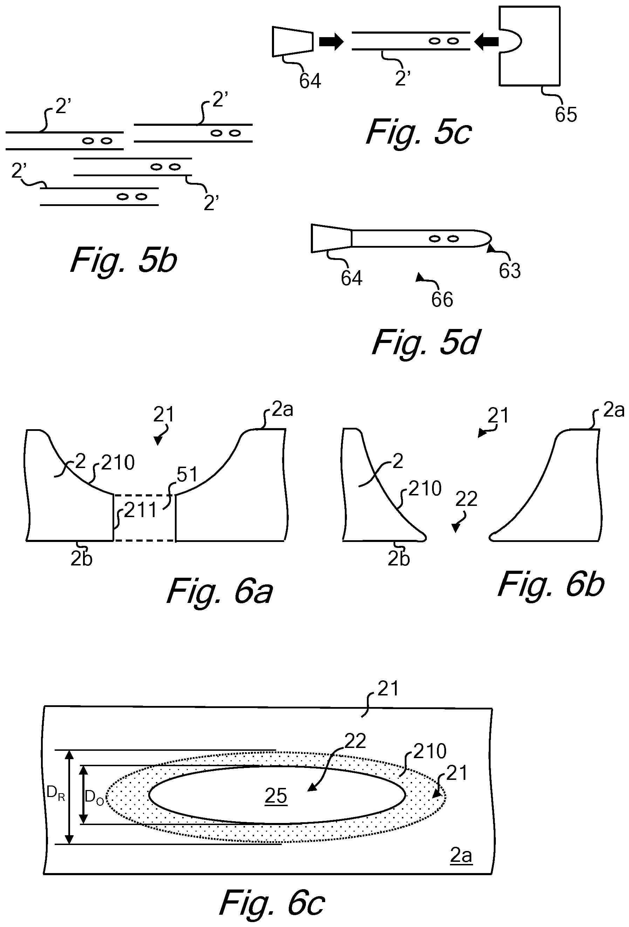

After steps S1-S5, the method may continue with steps S6-S8, illustrated schematically in figs. 5a-d. At step S6, the tubular member is cut into multiple catheter sections 2' shown in fig. 5a and fig. 5b. Each catheter section 2' comprises a recess and/or a recess with an opening into the central lumen. In some implementations the recess and/or recess with an opening is arranged in the same half portion of the catheter section, or in the same outermost third of the catheter section 2' or in the same outermost quarter of catheter section 2'. In either case, the at least one recess and/or recess with an opening is arranged closer to one of the two ends of the catheter section 2'. The closest end is referred to as the insertion end as it is this end which is first inserted through the urethra or rectum of a user when the catheter is in use. When a complete urinary catheter is to be manufactured each catheter section 2' may have a length corresponding to a typical urinary catheter, such as a length between 80 and 400 mm. Similarly, when a complete rectal catheter is to be manufactured each catheter section 2' may have a length corresponding to a typical rectal catheter, such as a length between 50 and 200 mm.

At step S7, a rounded tip 63 is formed at the insertion end, as seen in fig. 5c and fig. 5d. For example, the rounded tip 63 is formed by inserting the insertion end of the urinary or rectal catheter section 2' into a heated rounded mold 65 which melts and forms the tip to a rounded shape which makes the catheter more comfortable to introduce into the urethra or rectum. However, the rounded tip may also be formed using other techniques as such known for forming rounded catheter tips. For example, a rounded tip may be molded onto the end of the cut tubular member, or a rounded tip may be fastened to the end of the cut tubular member using an adhesive, laser welding or heat welding.

At step S8 a connector 64 is mounted to the outlet end of the catheter section 2'. The outlet end of the catheter section is opposite the insertion end. The connector may be configured to engage an extension tube, serve as a holding portion and/or serve as a spout to facilitate discharge of urine. In some exemplary implementations, the connector is attached using heat welding, using threads, solvent bonding, or using an adhesive to fixate the connector 64 onto the catheter section 2'. However it is also envisaged that the connector 64 can be formed as an integrated part of the catheter section wherein the catheter section is deformed to form the connector 64. In general steps S7 and S8 can be performed in any order, or simultaneously.

In some implementations, a hydrophilic coating is added to the tubular member 2 sometime after step S4. For example, it is envisaged that the rotatable dies only form recesses whereby the hydrophilic coating is added to the outside of the tubular member prior to forming the openings at step S5. This will ensure that the hydrophilic coating does not enter into the central lumen 25.

Additionally or alternatively, it is noted that step S5 involving forming an opening (which may occur at the same time as step S3 and S4) may be performed at any time after or during steps S6-S8. Especially, in implementations wherein the final catheter is coated with a hydrophilic coating layer which preferably is arranged only on the outside (and not inside) the central lumen of the catheter, step S5 may be performed separately from step S3 and S4, and after coating (e.g. step S5 is performed as a final step after step S8). Since the coating may be impractical for very long tubular members the coating process will typically be implemented after the cutting at step S6, and preferably also after steps S7 and/or S8 wherein step S5 is performed at any point in time after the coating.

Figs. 6a-c illustrate an exemplary recess 21 and opening 22 formed by rotating a rotatable die while pushing and removing a protrusion of the rotatable die into the tubular member. As seen in fig. 6a the resulting recess 21 may appear like a smooth concave indentation formed in the outer surface 2a. The recess 21 comprises a smooth recess surface 210 wherein at least a portion of the recess surface 210 has a surface normal that is different from a surface normal of the surrounding outer surface 2a. Fig. 6a also illustrates a region 51 which can be removed to form an opening inside the recess 21. Preferably, the recess 21 is larger, whereby the opening is smaller and arranged at a maximum depth inside the recess 21. This enables the smooth recess surfaces 210 to be arranged closest to the user when the tubular member 2 is used as a catheter, whereas any sharp edges formed at least in part by a surface 211 of the opening 22, are arranged deeper in the recess 21, at a place where they are not expected to come into contact with the user when the tubular member 2 is used as a catheter.

By altering e.g. the size and shape of the at least one protrusion on the rotatable die it is possible to form an opening 22 directly with the protrusion as shown in fig. 6b. Here, the protrusion has been pushed into, and removed from, the tubular member 2 to form a smoothly transitioning recess surface 210 with an opening 22 that leads all the way through the tubular member 2. Since the tubular member 2 at the time of removing the protrusion may still be heated, deformable and/or exhibiting a lower viscosity due to being ejected from the extrusion nozzle, any sharp edges which normally would arise during cutting, drilling, or milling are avoided since the material may (?) flow and deform smoothly.

Fig. 6c shows a view of the outer surface 2a in a region surrounding a recess 21 provided with an opening 22 formed in the tubular member 2. The recess 21 is arranged surrounding the opening 22 which communicates the central lumen 25 inside the tubular member 2. Preferably, the recess 21 is larger compared to the opening 22. For example, a dimension of the recess 21 measured along the outer surface 2a is at least 10% larger than a dimension of the opening 22, preferably at least 20% larger, more preferably at least 30% larger and most preferably at least 50% larger. That is, the outer dimension of the recess D_{R} may fulfill D_{R} ≥ 1.1D_{O}, D_{R} ≥ 1.2Dₒ, D_{R} ≥ 1.3Dₒ or D_{R} ≥ 1.5Dₒ wherein Dₒ is an outer dimension of the opening 22. The shape of the opening and/or recess may be chosen arbitrarily. For example, the opening is circular, elliptical (as shown in fig. 6c) or elongated (e.g. shaped like a slit).

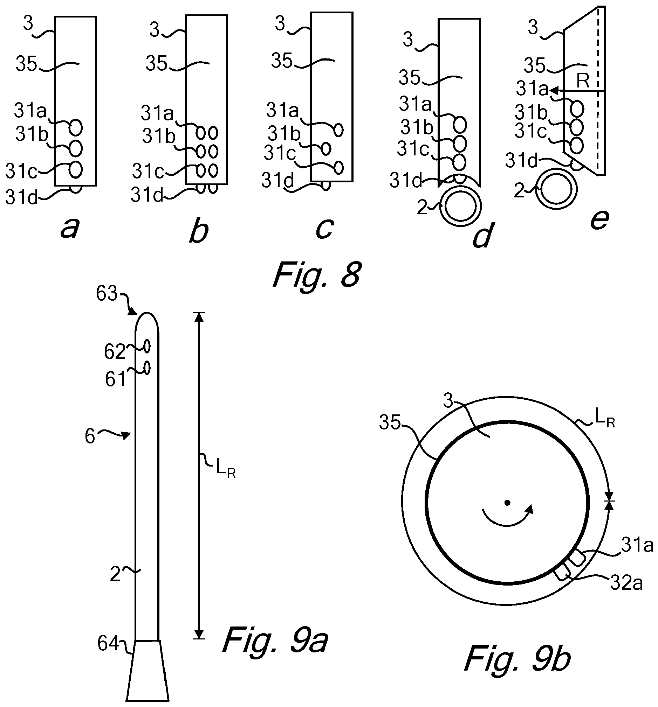

Fig. 8a-c show examples of how the one or more protrusions 31a-d can be arranged on a rotatable die 3. If more than one rotatable die 3 is used the arrangement of the one or more protrusions 31a-d on each rotatable die 3 may be the same or different. For example, a first rotatable die 3 may only have a single protrusion 31a-d whereas a second rotatable die has multiple protrusions 31a-d.

The rotatable die 3a in fig. 8a is substantially cylindrical and in this embodiment the rotatable die 3a is seen from along the longitudinal direction. The circumferential surface 35 is substantially flat wherein four protrusions 31a-d are arranged in a linear pattern with regular spacing. When the protrusions 31a-d are pushed into and removed from the tubular member 2 as the rotatable die rotates, they form a corresponding pattern of four recesses and/or openings.

The rotatable die 3 of fig. 8b features two columns of four protrusions each 31a-d for a total of eight protrusions 31a-d. When the protrusions 31a-d are pushed into and subsequently removed from the tubular member they form a corresponding pattern of two columns with four recesses and/or recesses provided with openings on the tubular member.

Arranging multiple protrusions 31a-d in one or more columns is merely exemplary, and it is understood that the protrusions 31a-d may be arranged in a zig-zag pattern as shown in fig. 8c, in a random pattern or other types of patterns. For example, the protrusions 31a-d may be arranged to form a distribution of recesses and/or openings that form number, a letter or a symbol.

While the circumferential surface 35 in the examples shown in fig. 8a-c is substantially flat this is merely exemplary. As shown in fig. 8d it is envisaged that the circumferential surface is curved, e.g. concave. For example, the curvature of the circumferential surface matches that of the tubular member 2, wherein the rotatable die 3 can be arranged very close to the tubular member 2.

Additionally or alternatively, the circumferential surface may be slanted with respect to the rotational axis R as shown in fig. 8e. A slanted circumferential surface 35 allows e.g. the protrusion(s) 31a-d to be pushed further down the west or east sides of the tubular member compared to a rotatable die having a circumferential surface substantially normal to the rotational axis R. For instance, two or more slanted rotatable dies 3 may be arranged close together to form a recess and/or recesses with openings in the tubular member that are arranged close together.

In some implementations, the at least one rotatable die 3 rotates around its rotational axis with an angular velocity ω that makes the tangential velocity at the circumferential surface 35 equal to the longitudinal speed at which the tubular member is extruded. Effectively, this means that the circumference of the rotatable die 3 will dictate the repetition distance for forming at least one recess and/or recess with opening in the tubular member as is illustrated in fig. 9a and fig. 9b.

Fig. 9a depicts schematically a urinary or rectal catheter 6 comprising a tubular member 2 extending from an insertion tip 63 to a connector/holding portion 64. As mentioned above, a urinary catheter is typically between 80 and 400 mm long whereby the tubular member 2, after extrusion and formation of the recess and/or recess with opening, is cut into 80 to 400 mm long pieces. Since rectal catheters are typically shorter (e.g. between 80 and 200 mm) the tubular member 2 is cut into shorter pieces to form rectal catheters.

Preferably, the recess(es) and/or recesses with opening(s) are therefore provided on the tubular member 2 with a repetition distance L_{R} substantially equal to a typical urinary or rectal catheter length. If the rotatable die keeps rotating with an angular velocity ω that makes the tangential velocity at the circumferential surface 35 match the extrusion speed, this repetition distance can be accomplished by selecting a rotatable die having a circumference equal to the repetition distance L_{R}, has shown in fig. 9b. In general, by providing N sets of at least one protrusion at N circumferentially equidistant positions the rotatable die can be realized with a circumference that is N times the repetition distance L_{R} while still the tangential velocity at the circumferential surface is equal to the extrusion speed and wherein N is an integer greater than or equal to 1. For example, since urinary catheters are between 80 and 400 mm long the diameter of the rotatable die may be between 25 mm and 125 mm (when N = 1), between 50 mm and 250 mm (when N = 2), between 150 mm and 375 mm (when N = 3) and so on.

However, since the rotatable die is preferably arranged as close as possible to the nozzle it may be more convenient to use as small rotatable die as possible whereby N = 1 or N = 2.

To ensure that the tangential speed of the rotatable die equals the extrusion speed the angular velocity ω of the rotatable die should be controlled such that ω*r = vₑ where r is the radius of the rotatable die and vₑ is the extrusion velocity. When the tangential speed of the rotatable die 3 equals the extrusion speed, it is envisaged that the rotatable die 3 remains in contact with the tubular member 2 constantly during extrusion. For example, it is envisaged that friction between the tubular member and the contacting rotatable die 3 contributes to driving the rotation of the rotatable die 3. To not deform the tubular member from its intended rounded shape (e.g. like a hollow cylinder with an oval, elliptical or circular base) it may be preferable if the circumferential surface of the rotatable die is concave, preferably with a radius of curvature matching that of the tubular member 2 (see e.g. fig. 8d).

It is also envisaged that it may not always be necessary to rotate the rotatable die 3 with an angular velocity ω that causes the tangential velocity at the circumferential surface of the rotatable die to match the extrusion velocity. For example, the rotatable die 3 may rotate with a tangential velocity at a speed which is greater than or smaller than the extrusion speed and/or with a tangential velocity which varies over time.

In fig. 10, a manufacturing system according to some implementations is shown with a rotatable die 3 which is linearly displaceable between an engagement position (the current position of the rotatable die 3 as shown in fig. 8) and a disengagement position 3'. The engagement position and the disengagement position are separated along the transverse axis T such that the rotatable die 3 is closer to the tubular member 2 at the engagement position compared to at the disengagement position 3'. At the disengagement position 3' the at least one protrusion 31, 32 and the circumferential surface 35 is freed from the tubular member 2 and the rotatable die 3 can rotate at any speed, and in any direction, without interacting with the tubular member 2. Accordingly, the rotatable die can be normally in the disengaged position 3' whereby the rotatable die is moved to the engagement position to form the recesses and/or the recesses with openings 22 in the tubular member 2. This allows the rotatable die 3 to move with any rotational speed, or even in reverse, at the disengaged position while moving with the extrusion speed at the engagement position.

In one example, the rotatable die rotates counterclockwise (i.e. along the extrusion direction) in the engaged position, while the protrusion(s) are pushed into and removed from the tubular member. The rotatable die is then moved to the disengaged position, whereafter the rotary die rotates clockwise or counterclockwise to revert to a position with the protrusions ready to be pushed into the tubular member by counterclockwise rotation. With a linearly displaceable rotatable die it is not necessary that the rotatable die is circular, it may e.g. be shaped like an ellipse or shaped like a circle sector with the protrusion(s) being arranged on the circle arc of the circle sector.

Fig. 11 shows another exemplary manufacturing system with a rotatable die having a circumference which is smaller than the repetition distance L_{D}. It is understood that by providing a rotatable die with protrusion(s) that extend sufficiently far from the circumferential surface 35 the rotatable die 3 will only contact the tubular member when the protrusion(s) are pushed into the tubular member. In fact, the circumferential surface 35 may in some implementations normally not contact the tubular member 2 at all, whereby the rotatable die is rotated by a drive unit.

After the protrusion(s) 31, 32 have been removed from the tubular member 2 the rotatable die 3 no longer contacts the tubular member 2 whereby it can be rotated counterclockwise to revert to a position in which it is ready to push in the protrusion(s) again. To enable a repetition distance greater than the circumference of the rotatable die 3 the rotatable die 3 rotates with an angular velocity associated with a tangential velocity at the protrusion(s) which is lower than the extrusion speed. However it is still possible for the tangential velocity of the rotatable die 3 to match the extrusion velocity while the protrusion is pushed into and removed from the tubular member. That is, the angular velocity of the rotatable die 3 may be variable over time to enable a repetition distance that is greater than or smaller than the circumference of the rotatable die 3.

Fig. 12 illustrates how more than one rotatable die 3a-d may be arranged around the tubular member 2 to form openings and/or recesses 21 in different angular sectors of the tubular member 2. In fig. 12 four rotatable dies 3a-d are arranged in a north, east, south, and west arrangement with each rotatable die having at least one protrusion 31 forming a corresponding recess in the outer surface of the tubular member 2. In general two or three rotatable dies, or more than four rotatable dies, may be arranged arbitrarily around the tubular member 2 coming out of the nozzle, depending on the desired pattern of recesses and/or recesses with openings in the final urinary or rectal catheter.

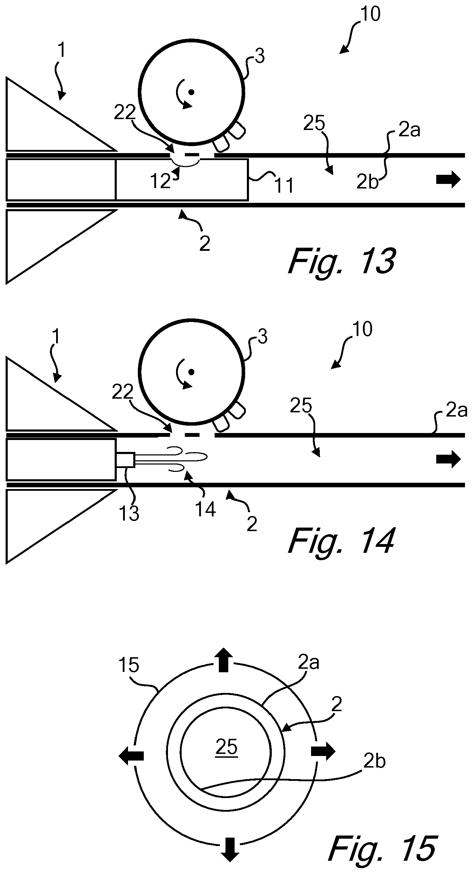

Fig. 13 shows an optional support structure 11 that may be provided in the manufacturing system 10. The support structure 11 extends inside the central lumen 25 to the position downstream of the nozzle 1 where the protrusion(s) of the rotatable die 3 will be introduced into the tubular member 2. The cross-section of the support structure 11, when seen along the longitudinal direction L, is substantially equal to that of the central lumen 25. Hereby, the support structure 11 helps maintain the shape of the tubular member 2, and especially the central lumen 25, even when the protrusion(s) of the rotatable die 3 press onto, and partially or fully through, the tubular member 2. Depending on factors such as the material properties of the liquid material 41, the dimensions of the tubular member 2 and the size of the protrusions, there is a risk that the tubular member 2 collapses due to the force exerted by the protrusion(s) when they come into contact with the outer surface 2a. The support structure 11 mitigates this risk by occupying the space inside the central lumen 25 and supporting the inner surface 2b of the central lumen 25.

In some implementations, the support structure 11 further comprises at least one recess 12 facing the of the rotatable die 3. The at least one recess allows the protrusion(s) to penetrate all the way through the tubular member 2 (to form an opening 22) without the support structure 11 obstructing the rotational movement of the rotatable die 3.

In some implementations, the support structure 11 is fixated to, or formed integrally with, the nozzle 1. For example, as described above the outlet of the nozzle 1 may be ring shaped whereby the support structure 11 is fixated to or extends from a central region inside the ring shaped outlet.

Fig. 14 depicts a manufacturing system having an inlet 13 for injection of a fluid 14 (typically a gas, such as air or nitrogen) into the central lumen 25 of the tubular member. By injecting a fluid 14 into the central lumen 25 a positive pressure can be provided inside the central lumen 25 which adds rigidity the tubular member 2 and prevents it from collapsing during e.g. pushing of the protrusion(s) of the rotatable die(s) 3 into the tubular member 2. Injection of fluid 14 may be used as an alternative to, or in addition to, the support structure 11 shown in fig. 13. For example, it is envisaged that is some embodiments the fluid 14 is injected downstream of the support structure, whereby the support structure adds support at the position where protrusions are pushed into the tubular member 2 and the fluid supports 14 the tubular member downstream of the support structure 11 to support the tubular member 2 as it cools and/or cures.

The free end of the tubular member 2, that is the end opposite the nozzle outlet, defining the extrusion end of the tubular member 2 may be closed (e.g. with a cap, molten or cured material from the extrusion process, or a gripper pulling the tubular member 2 along the longitudinal direction) whereby the injected fluid 14 becomes trapped in the central lumen 25 and can be used to build up a positive pressure inside the central lumen 25 to provide rigidity to the tubular member 2. When only recesses, and no openings, are provided by the rotatable die(s) 3 the central lumen 25 may form a completely sealed chamber during extrusion.

On the other hand, when one or more openings 22 are formed directly by the one rotatable die(s) 3 the injected fluid 14 may leak out via the openings. However, since the openings tend to be small, a local positive pressure can still be maintained inside the central lumen 25 to add rigidity to the tubular member 2.

Optionally, the injected fluid 14 (typically realized with a gas, such as air or nitrogen) is heated or cooled prior to injection. Injecting cooled fluid may facilitate more rapid solidifying. On the other hand, injecting heated fluid may heat the tubular member such that it remains molten or remains at a temperature with lower viscosity for a longer period of time, which may facilitate forming smooth edges.

Fig. 15 shows another example of an arrangement that can be used to make sure that the tubular member 2 retains its shape. The tubular member 2 is inserted into a tube-shaped chamber 15 in which a negative pressure is maintained. For example, air is actively pumped out of the tube shaped chamber 15. The negative pressure around the tubular member will pull the tubular member 2 outwards, causing the tubular member to retain its rounded shape. Put differently, the pressure differential between the inside of the central lumen 25 and the outside of the tubular member 2 will cause the higher pressure inside the central lumen to push outwardly on the inner surface 2b of the tubular member 2, whereby the tubular member becomes supported and does not collapse. Similar to when a fluid is injected into the central lumen 25, the negative pressure tube shaped channel may be used even when the tubular member 2 has one or more openings into the central lumen. While each opening may cause some fluid leakage and local reduction in pressure differential the leakage may be small and/or counter acted such that the tubular member 2 is at least locally supported by the pressure differential.

While e.g. figs. 1 and 2 depict the extrusion process with the longitudinal direction pointing seemingly horizontally it is envisaged that this is merely exemplary and in practice the longitudinal direction may be angled with respect to a horizontal ground plane, e.g. pointing vertically upwards or vertically downwards. In fact, in some implementations it may be preferable if the extrusion direction points e.g. vertically downwards to enable gravity assisted extrusion. This may allow much higher extrusion temperatures to be used wherein the extruded tubular member e.g. still is in its melted state when the at least one protrusion 31a, 32a is pushed into the tubular member.

## Claims

1. A method for manufacturing catheter tubing for a urinary catheter or a rectal catheter, comprising:
extruding a tubular member by passing a liquid material through a nozzle, the tubular member comprising a rounded outer surface and a central lumen, wherein the tubular member is extruded in a longitudinal direction;
rotating a first rotatable die around a rotation axis arranged at a longitudinal position downstream of the nozzle, wherein said first rotatable die comprises at least one protrusion;
while said first rotatable die is rotating:
pushing said at least one protrusion of the first rotatable die into the tubular member at least a portion of the distance between the outer surface and the central lumen; and
removing the at least one protrusion from the tubular member, such that the at least one protrusion leaves at least one recess or opening in the tubular member.

2. The method for producing catheter tubing according to claim 1, comprising:
pushing said at least one protrusion of the first rotatable die into the tubular member a portion of the distance between the outer surface and the at least one central lumen;
removing the at least one protrusion from the tubular member, such that the at least one protrusion leaves a recess in the outer surface of the tubular member; and
providing an opening inside the recess, the opening extending to communicate with the central lumen.

3. The method for producing catheter tubing according to claim 1, comprising:
pushing the at least one protrusion of the first rotatable die into the tubular member the full distance between the outer surface and the at least one central lumen; and
removing the at least one protrusion from the tubular member, such that the protrusion leaves an opening in the tubular member, said opening communicating with the central lumen.

4. The method for producing catheter tubing according to any of the preceding claims, further comprising:
rotating a second rotatable die around a second rotation axis arranged at a second longitudinal position downstream of the nozzle, wherein said second rotatable die comprises at least one protrusion;
while said second rotatable die is rotating:
pushing said at least one protrusion of the second rotatable die into the tubular member at least a portion of the distance between the outer surface and the central lumen; and
removing the at least one protrusion of the second rotatable die from the tubular member, such that each at least one protrusion leaves at least one second recess or opening in the tubular member.

5. The method for producing catheter tubing according to claim 4, wherein the first and second rotatable dies are arranged on opposite sides of the tubular member such that the first recess or opening and second recess or opening are formed on opposite sides of the tubular member.

6. The method for producing catheter tubing according to any of the preceding claims, further comprising:
passing the tubular member over a support structure, the support structure being arranged in the central lumen and extending at least to the first longitudinal position downstream of the nozzle where the protrusion is pushed into the tubular member.

7. The method for producing catheter tubing according to any of the preceding, further comprising:
injecting a fluid inside the central lumen of the tubular member to increase the pressure inside the central lumen.

8. The method for producing catheter tubing according to any of the preceding claims, further comprising:
downstream of each rotatable die, inserting the tubular member into a tube shaped chamber, the tube shaped chamber having an inner diameter which is larger than an outer diameter of the tubular member; and
providing a negative pressure inside the tube shaped chamber to exert an expanding force on the tubular member.

9. The method for producing catheter tubing according to any of the preceding claims, wherein the first rotatable die is circular and has a circumference equaling a repetition length for the forming of the at least one recess or opening, the method further comprising:
rotating the first rotatable die such that a tangential velocity at the periphery of the first rotatable die equals an extrusion velocity of the tubular member.

10. The method for producing catheter tubing according to any of the preceding claims, wherein at least the first rotatable die is linearly displaceable at least along a transverse axis perpendicular to the longitudinal axis, the method further comprising:
moving the first rotatable die transversely towards the tubular member, prior to or during the pushing of the at least one protrusion of the first rotatable die into the tubular member; and
moving the first rotatable die transversely away from the tubular member, during or after removal of the at least one protrusion of the first rotatable die from the tubular member.

11. The method for producing catheter tubing according to any of the preceding claims, wherein said first rotatable die comprises a plurality of protrusions forming a plurality of recesses and/or openings.

12. The method for producing catheter tubing according to claim 11, wherein the plurality of recesses and/or openings are distributed over a longitudinal distance that is smaller than 10 cm, smaller than 8 cm or smaller than 6 cm.

13. The method for producing catheter tubing according to any of the preceding claims, further comprising:
cutting the tubular member into a tubular member section, the section having at least one opening or recess; and
forming a closed tip at one end of the tubular member section.

14. The method for producing catheter tubing according to any of the preceding claims,
wherein the catheter is a urinary catheter, and wherein an outer diameter of the tubular member is smaller than 10 mm, preferably smaller than 8 mm and most preferably smaller than 7 mm, or
wherein the catheter is a rectal catheter, and wherein an outer diameter of the tubular member is at least 5 mm, at least 10 mm or at least 12 mm, preferably between 5 mm and 15 mm.

15. A system for manufacturing catheter tubing for urinary catheters or rectal catheters, the manufacturing system comprising:
a nozzle, configured to extrude a liquid material to form a tubular member having a rounded outer surface and a central lumen, wherein the tubular member is extruded in a longitudinal direction, and
a first rotatable die, the first rotatable die comprising a smooth cylindrical peripheral surface and at least one protrusion arranged on the peripheral surface,
wherein the rotatable die is configured to rotate around a rotational axis at a longitudinal position downstream of the nozzle, and
wherein all protrusions are confined to a region covering at most 20% of the peripheral surface.
